(19) **Europäisches Patentamt**
**European Patent Office**
**Office européen des brevets**

(11) **EP 1 177 319 B1**

(12) **EUROPÄISCHE PATENTSCHRIFT**

(45) Veröffentlichungstag und Bekanntmachung des Hinweises auf die Patenterteilung:
**04.07.2007 Patentblatt 2007/27**

(51) Int Cl.:
***C12Q 1/68*** (2006.01)

(21) Anmeldenummer: **00931109.3**

(86) Internationale Anmeldenummer:
**PCT/EP2000/003989**

(22) Anmeldetag: **04.05.2000**

(87) Internationale Veröffentlichungsnummer:
**WO 2000/068421 (16.11.2000 Gazette 2000/46)**

(54) **VERFAHREN ZUM NACHWEISEN VON MIKROORGANISMEN IN EINER PROBE**

METHOD FOR DETECTING MICROORGANISMS IN A SAMPLE

PROCEDE DE DETECTION DE MICRO-ORGANISMES DANS UN ECHANTILLON

(84) Benannte Vertragsstaaten:
**AT BE CH CY DE DK ES FI FR GB GR IE IT LI LU MC NL PT SE**

(30) Priorität: **07.05.1999 DE 19921281**
**05.08.1999 DE 19936875**

(43) Veröffentlichungstag der Anmeldung:
**06.02.2002 Patentblatt 2002/06**

(73) Patentinhaber: **Vermicon AG**
**80992 München (DE)**

(72) Erfinder: **SNAIDR, Jiri**
**D-80992 München (DE)**

(74) Vertreter: **Bohmann, Armin K.**
**Bohmann & Loosen**
**Anwaltssozietät**
**Nymphenburger Strasse 1**
**80335 München (DE)**

(56) Entgegenhaltungen:
**WO-A-98/15656          WO-A-99/18234**
**DE-A- 4 216 949        US-A- 5 750 340**

- **SAMBROOK, FRITSCH, AND MANIATIS: "Molecular cloning-A laboratory manual" 1987 , COLD SPRING HARBOUR PRESS , US XP002162309 "Hybridization of radiolabeled probes to immobilized nucleic acids" pgs 9.47-9.55 Seite 9.51 Seite 9.55**
- **NORDENTOFT STEEN ET AL: "Evaluation of a fluorescence-labelled oligonucleotide probe targeting 23S rRNA for in situ detection of Salmonella serovars in paraffin-embedded tissue sections and their rapid identification in bacterial smears." JOURNAL OF CLINICAL MICROBIOLOGY, Bd. 35, Nr. 10, 1997, Seiten 2642-2648, XP002162308 ISSN: 0095-1137**
- **MOBARRY B K ET AL: "PHYLOGENETIC PROBES FOR ANALYZING ABUNDANCE AND SPATIAL ORGANIZATION OF NITRIFYING BACTERIA" APPLIED AND ENVIRONMENTAL MICROBIOLOGY,US,WASHINGTON,DC, Bd. 62, Nr. 6, 1. Juni 1996 (1996-06-01), Seiten 2156-2162, XP002068770 ISSN: 0099-2240**
- **AMANN R I ET AL: "PHYLOGENETIC IDENTIFICATION AND IN SITU DETECTION OF INDIVIDUAL MICROBIAL CELLS WITHOUT CULTIVATION" MICROBIOLOGICAL REVIEWS, US,AMERICAN SOCIETY FOR MICROBIOLOGY, WASHINGTON, DC, Bd. 59, Nr. 1, 1. März 1995 (1995-03-01), Seiten 143-169, XP002026194 ISSN: 0146-0749**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**SEQUENZPROTOKOLL/V7231**

(1) ALLGEMEINE ANGABEN:

(i) ANMELDER:

(A) NAME: VERMICON AG
(B) STRASSE: Dachauer Str. 148
(C) ORT: München
(E) LAND: Deutschland
(F) POSTLEITZAHL: 80992

(ii) BEZEICHNUNG DER ERFINDUNG: Verfahren zum Nachweisen von Mikroorganismen in einer Probe

(iii) ANZAHL DER SEQUENZEN: 8

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(v) DATEN DER JETZIGER ANMELDUNG: ANMELDENUMMER: DE 199 36 875.9

(2) ANGABEN ZU SEQ ID NO: 1:

(i) SEQUENZKENNZEICHEN:

(A) LANGE: 18 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 1:
TCGACTGACT TCAGCTCC          18

(2) ANGABEN ZU SEQ ID NO: 2:

(i) SEQUENZKENNZEICHEN:

(A) LÄNGE: 18 Basenpaare

          (B) ART: Nucleotid
          (C) STRANGFORM: Einzelstrang
          (D) TOPOLOGIE: linear

     (ii) ART DES MOLEKÜLS: cDNA

     (xi) SEQUENZBESCHREIBUNG: SEQ ID NO: 2:
     GCTAACTACT TCTGGAGC          18

(2) ANGABEN ZU SEQ ID NO: 3:

     (i) SEQUENZKENNZEICHEN:

          (A) LÄNGE: 18 Basenpaare
          (B) ART: Nucleotid
          (C) STRANGFORM: Einzelstrang
          (D) TOPOLOGIE: linear

     (ii) ART DES MOLEKÜLS: cDNA
     (xi) SEQUENZBESCHREIBUNG: SEQ ID NO: 3:
     ACCCTCCTCT CCCGGTCT          18

(2) ANGABEN ZU SEQ ID NO: 4:

     (i) SEQUENZKENNZEICHEN:

          (A) LÄNGE: 18 Basenpaare
          (B) ART: Nucleotid
          (C) STRANGFORM: Einzelstrang
          (D) TOPOLOGIE: linear

     (ii) ART DES MOLEKÜLS: cDNA
     (xi) SEQUENZBESCHREIBUNG: SEQ ID NO: 4:
     TGGCCCACCG GCTTCGGG          18

(2) ANGABEN ZU SEQ ID NO: 5:

     (i) SEQUENZKENNZEICHEN:

          (A) LÄNGE: 18 Basenpaare
          (B) ART: Nucleotid
          (C) STRANGFORM: Einzelstrang
          (D) TOPOLOGIE: linear

     (ii) ART DES MOLEKÜLS: cDNA
     (xi) SEQUENZBESCHREIBUNG: SEQ ID NO: 5:
     GCTAACTACT TCTGGAGC          18

(2) ANGABEN ZU SEQ ID NO: 6:

     (i) SEQUENZKENNZEICHEN:

          (A) LÄNGE: 18 Basenpaare
          (B) ART: Nucleotid
          (C) STRANGFORM: Einzelstrang
          (D) TOPOLOGIE: linear

     (ii) ART DES MOLEKÜLS: cDNA

(xi) SEQUENZBESCHREIBUNG: SEQ ID NO: 6:
GCTGCCTCCC GTAGGAGT          18

(2) ANGABEN ZU SEQ ID NO: 7:

(i) SEQUENZKENNZEICHEN:

(A) LÄNGE: 18 Basenpaare
(B) ART: Nucleotid
(C) STRANGFORM: Einzelstrang
(D) TOPOLOGIE: linear

(ii) ART DES MOLEKÜLS: cDNA
(xi) SEQUENZBESCHREIBUNG: SEQ ID NO: 7:
GCAGCCACCC GTAGGTGT          18

(2) ANGABEN ZU SEQ ID NO: 8:

(i) SEQUENZKENNZEICHEN:

(A) LÄNGE: 18 Basenpaare
(B) ART: Nucleotid
(C) STRANGFORM: Einzelstrang
(D) TOPOLOGIE: linear

(ii) ART DES MOLEKÜLS: cDNA
(xi) SEQUENZBESCHREIBUNG: SEQ ID NO: 8:
GCTGCCACCC GTAGGTGT          18

**Beschreibung**

Die Identifizierung von Mikroorganismen konnte viele Jahrzehnte nur nach vorhergehender Kultivierung der Mikroorganismen und damit einhergehender Amplifizierung erfolgen. Diese Kultivierung erfolgt z.B. für Viren auf ihrem jeweiligen Wirtsorganismus, für Bakterien, Pilze und einzellige Algen in Nährmedien. Für die Erfassung der Anzahl lebensfähiger Mikroorganismen in einer bestimmten Probe wurden Medien bzw. Bedingungen gewählt, welche eine selektive Erfassung bestimmter Gruppen weitgehend ausschließen sollten. So wurden z.B. von bakteriellen Einzelkolonien Reinkulturen angelegt und diese aufgrund phänotypischer Merkmale, z.B. ihrer Morphologie und Stoffwechselwege, identifiziert. Die Identifizierung von Mikroorganismen nach vorheriger Kultivierung ist jedoch mit zwei entscheidenden Nachteilen verbunden. Erstens belegen Untersuchungen an den verschiedensten Umweltproben, daß nur 0,1 bis 14 % aller Bakterien z.Z. kultivierbar sind. Zweitens konnte bewiesen werden, daß bei der Kultivierung starke Populationsverschiebungen auftreten können, d.h. bestimmte Bakteriengruppen im Labor bevorzugt bzw. andere diskriminiert werden.

Dies bedeutet nicht nur, daß ein Großteil der Bakterien in Umweltproben nicht erkannt werden kann, sondern auch, daß diejenigen Bakterien, welche identifiziert werden, meist ein verzerrtes Abbild der wahren Populationsstrukturen darstellen. Fehleinschätzungen der Populationsverhältnisse in bezug auf Identifizierung und Quantifizierung der Bakterien sind die Folge.

Anfang der neunziger Jahre wurde ein Verfahren zur in situ-Hybridisierung mit fluoreszenzmarkierten Oligonukleotidsonden entwickelt, das in vielen Umweltproben erfolgreich zum Einsatz kam (Amann et al. (1990) J. Bacteriol. 172: 762). Dieses "FISH" genannte (fluoreszierende in situ-Hybridisierung) Verfahren beruht auf der Tatsache, daß die in jeder Zelle vorhandenen ribosomalen RNAs (rRNAs) hochkonservierte, d.h. wenig spezifische, und weniger konservierte, d.h. gattungs- und artspezifische Sequenzen umfaßt. Schon Mitte der achtziger Jahre wurde gezeigt, daß die Sequenzen der 16S- und 23S-rRNA zur Identifizierung von Mikroorganismen genutzt werden können (Woese (1987) Microbiol. Reviews 51:221; De Long et al. (1989) Science 243:1360). Bei dem FISH-Verfahren werden fluoreszenzmarkierte Gensonden, deren Sequenzen zu einer bestimmten Region auf der ribosomalen Zielsequenz komplementär sind, in die Zelle geschleust. Die Sondenmoleküle sind in der Regel 16 bis 20 Basen lange, einzelsträngige Desoxyribonukleinsäurestücke und sind einem Zielbereich komplementär, der für eine bestimmte Bakterienart oder eine bakterielle Gattung spezifisch ist. Findet die fluoreszenzmarkierte Gensonde in einer Bakterienzelle ihre Zielsequenz, so bindet sie daran und die Zellen können aufgrund ihrer Fluoreszenz im Fluoreszenzmikroskop detektiert werden.

Es konnte gezeigt werden, daß durch die in situ-Hybridisierung mit fluoreszenzmarkierten Sonden bis zu 90% einer Bakteriengesamtpopulation detektiert werden können. Das Verfahren stellt daher bereits eine wesentliche Verbesserung gegenüber dem Stand der Technik dar, der die Detektion von maximal 14% der Bakterienpopulation einer Umweltprobe möglich machte. Darüber hinaus erlaubt es das Verfahren der in situ-Hybridisierung mit fluoreszenzmarkierten Sonden, den aktiven Anteil einer Population zu bestimmen, indem das Verhältnis einer gegen alle Bakterien gerichteten Sonde und dem Trockengewicht bestimmt wird. Schließlich erlaubt das Verfahren, Bakterien direkt am Ort ihres Wirkens sichtbar zu machen, wodurch Wechselwirkungen zwischen verschiedenen Bakterienpopulation erkannt und analysiert werden können.

Innerhalb der letzten Jahre wurde die Technik der in situ-Hybridisierung mit fluoreszenzmarkierten Sonden für die verschiedensten Umweltproben ausgetestet und erfolgreich angewandt. So konnten durch den Einsatz von Gensonden im Boden, in Protozoen, in Biofilmen, in der Luft, in Seen, in biologisch aktivierten Filtern und im Abwasser von Kläranlagen die jeweiligen Bakterienpopulationen untersucht und neuartige Bakterien identifiziert werden. Der Schwerpunkt lag hierbei in der Analyse der Bakterienpopulationen bei der Abwasserreinigung. Tropfkörper-, Raumfiltrations- und Belebtschlammanlagen wurden ebenso untersucht wie die Nachklärbecken und entsprechenden Vorfluter (Snaidr et al. (1997) Appl. Environ. Microbiol. 63:2884). Durch die in situ-Hybridisierungstechnik konnte gezeigt werden, daß es bei der Erfassung der Belebtschlammflora durch Kultivierung zu einem Kultivierungsshift kommt (Wagner et al. (1993) Appl. Environ. Microbiol. 59:1520). Kultivierungsabhängige Verfahren liefern daher nur einen sehr verfälschten Einblick in die Zusammensetzung und Dynamik der mikrobiellen Biozönose. Durch diese Medium-abhängige Verzerrung der realen Verhältnisse innerhalb der Bakterienpopulation werden Bakterien, die im Belebtschlamm eine untergeordnete Rolle spielen, aber den eingesetzten Kultivierungsbedingungen gut angepaßt sind, in ihrer Bedeutung dramatisch überschätzt. So konnte gezeigt werden, daß aufgrund eines solchen Kultivierungsartefakts die Bakteriengattung *Acinetobacter* bezüglich ihrer Rolle als biologischer Phosphatentferner in der Abwasserreinigung völlig falsch eingeschätzt wurde.

Obwohl die in situ-Hybridisierung mit den neu entwickelten fluoreszenzmarkierten Gensonden eine rasche und genaue Analyse von Bakterienpopulationen im Abwasser möglich macht, konnte sie sich in der Praxis noch nicht durchsetzen. Gründe hierfür sind der hohe Anschaffungspreis für die benötigten technischen Geräte wie Fluoreszenzmikroskope, der Bedarf an qualifizierten Kräften, welche für die Durchführung und Auswertung zur Verfügung stehen müssen, sowie die daraus resultierende geringe Anzahl möglicher Referenzmessungen. Weiterhin ist ein hoher Zeitaufwand für das Auszählen der detektierten Bakterienpopulationen (Quantifizierung) notwendig. Überdies erfordert das Auszählen hohe Erfahrungswerte, da zwischen echten (Sondenbindung hat tatsächlich stattgefunden) und falschen Signalen (Autofluoreszenz, keine Zellen) unterschieden werden muß.

Aufgabe der vorliegenden Erfindung ist es daher, ein Verfahren zur Verfügung zu stellen, mit dem Mikroorganismen möglichst ohne vorherige Kultivierung nachgewiesen und gegebenenfalls quantifiziert werden können.

Eine weitere wesentliche Aufgabe der vorliegenden Erfindung ist es, ein Verfahren zum Nachweis von Mikroorganismen bereitzustellen, das unter Bedingungen durchgeführt wird, die gewährleisten, daß die Messergebnisse bei der Detektion und, falls erwünscht, bei der Quantifizierung nicht nachteilig beeinflußt werden.

Ferner ist es eine Aufgabe der vorliegenden Erfindung, ein Verfahren zum Nachweis von Mikroorganismen bereitzustellen, das umweltfreundlicher, schneller durchführbar und leichter handhabbar ist als die im Stand der Technik beschriebenen Verfahren.

Erfindungsgemäß werden diese Aufgaben durch ein Verfahren zum Nachweis von Mikroorganismen in einer Probe mittels einer Nukleinsäuresonde gelöst, wobei das Verfahren die folgenden Schritte umfaßt:

a) Fixieren der in der Probe enthaltenen Mikroorganismen;

b) Inkubieren der fixierten Mikroorganismen mit nachweisbaren Nukleinsäuresondenmolekülen, um eine Hybridisierung herbeizuführen;

c) Entfernen nicht hybridisierter Nukleinsäuresondenmoleküle;

d) Ablösen der hybridisierten Nukleinsäuresondenmoleküle und

e) Detektieren und gegebenenfalls Quantifizieren der abgelösten Nukleinsäuresondenmoleküle.

Im Rahmen der vorliegenden Erfindung soll unter "Fixieren" von Mikroorganismen eine Behandlung verstanden werden, mit der die den jeweiligen Mikroorganismus umgebenden Hüllen so durchlässig gemacht werden sollen, daß die Nukleinsäuresonde mit der gegebenenfalls kovalent verbundenen Markierung durch die Hülle penetrieren kann, um so die Zielsequenzen im Zellinneren erreichen zu können. Bei der Hülle kann es sich z.B. um die ein Virus umgebende Lipidhülle, um die Zellwand eines Bakteriums oder die Zellmembran eines einzelligen Mikroorganismus handeln. Zur Fixierung wird üblicherweise eine geringprozentige Paraformaldehydlösung verwendet. Sollte mit einer Paraformaldehydlösung im Einzelfall die einen Mikroorganismus umgebende Schutzhülle nicht penetrierbar gemacht werden können, so sind dem Fachmann ausreichend weitere Maßnahmen bekannt, die zu demselben Ergebnis führen. Dazu zählen beispielsweise Ethanol, Methanol, Mischungen dieser Alkohole mit Paraformaldehyd, enzymatische Behandlungen, Ultraschallbehandlung etc.

Bei einer Nukleinsäuresonde im Sinne der Erfindung kann es sich um eine DNA-oder RNA-Sonde handeln, die in der Regel zwischen 12 und 1000 Nukleotide umfassen wird, bevorzugt zwischen 12 und 100 oder 15 und 50, besonders bevorzugt zwischen 17 und 25 Nukleotide. Die Nukleinsäuresonde ist so ausgewählt, daß es eine zu ihr komplementäre Sequenz im nachzuweisenden Mikroorganismus bzw. in der Gruppe nachzuweisender Mikroorganismen gibt. Komplementarität muß bei einer Sonde von nur ca. 15 Nukleotiden über 100 % der Sequenz gegeben sein, bei Oligonukleotiden mit mehr als 15 Nukleotiden sind ein bis mehrere Fehlpaarungsstellen erlaubt. Es muß jedoch gewährleistet sein, daß das Nukleinsäuresondenmolekül mit moderaten und/oder stringenten Hybridisierungsbedingungen tatsächlich mit der Zielsequenz hybridisiert. Moderate Bedingungen im Sinne der Erfindung sind z.B. 0 % Formamid in einem Hybridisierungspuffer wie er in Beispiel 1 beschrieben ist. Stringente Bedingungen im Sinne der Erfindung sind beispielsweise 20 - 80 % Formamid in dem in Punkt 5.2 von Beispiel 1 beschriebenen Puffer.

Die Dauer der Hybridisierung beträgt üblicherweise zwischen 10 Minuten und 12 Stunden; bevorzugt erfolgt die Hybridisierung für etwa 2 Stunden. Die Hybridisierungstemperatur beträgt bevorzugt zwischen 44°C und 48°C, besonders bevorzugt 46°C, wobei der Parameter der Hybridisierungstemperatur, wie auch die Konzentration an Salzen und Detergentien in der Hybridisierungslösung in Abhängigkeit von der Sonden bzw. den Sonden, insbesondere deren Länge(n) und dem Grad der Komplementarität mit der Targetsequenz in der nachzuweisenden Zelle optimiert werden kann. Der Fachmann ist mit hier einschlägigen Berechnungen vertraut.

Im Rahmen des erfindungsgemäßen Verfahrens hat eine typische Hybridisierungslösung eine Salzkonzentration von 0,1 M bis 1,5 M, bevorzugt von 0,9 M, wobei es sich bei dem Salz vorzugsweise um Natriumchlorid handelt. Weiter umfaßt der Hybridisierungspuffer üblicherweise ein Detergens, wie z.B. Natriumdodecylsulfat (SDS), in einer Konzentration von 0,001-0,1 %, bevorzugt in einer Konzentration von 0,01 %, und Tris/HCl in einem Konzentrationsbereich von 0,001-0,1 M, vorzugsweise in einer Konzentration von 0,02 M. Der pH-Wert von Tris/HCl beträgt üblicherweise zwischen 6 und 10, wobei ein pH von ca. 8,0 bevorzugt ist. Wie oben erwähnt, kann die Hybridisierungslösung des weiteren zwischen 0% und 80% Formamid enthalten, je nachdem welcher Grad von Stringenz erwünscht ist bzw. benötigt wird.

Die Nukleinsäuresonde sollte im Hybridisierungspuffer wenn möglich in einer Menge von 15 ng bis 1000 ng anwesend sein, wobei diese Menge vorzugsweise in 80 $\mu$l Hybridisierungslösung enthalten ist. Besonders bevorzugt beträgt die Sondenkonzentration 125 ng/80 $\mu$l Hybridisierungslösung.

Nach erfolgter Hybridisierung sollten die nicht hybridisierten und überschüssigen Sondenmoleküle entfernt werden, was üblicherweise mittels einer herkömmlichen Waschlösung bzw. Waschpuffers erfolgt. Dieser Waschpuffer enthält gewöhnlicherweise 0,001-0,1% eines Detergens wie SDS, wobei eine Konzentration von 0,01% bevorzugt wird, und Tris/HCl in einer Konzentration von 0,001-0,1 M, bevorzugt 0,02 M, wobei der pH-Wert von Tris/HCl im Bereich von 6,0 bis 10,0, vorzugsweise bei 8,0, liegt. Weiter enthält der Waschpuffer üblicherweise NaCl, wobei die Konzentration je nach benötigter Stringenz zwischen 0,003 M und 0,9 M, bevorzugt zwischen 0,01 M und 0,9 M, beträgt. Zusätzlich kann die Waschlösung EDTA enthalten, wobei die Konzentration vorzugsweise 0,005 M beträgt.

Das "Abwaschen" der nicht gebundene Sondenmoleküle erfolgt üblicherweise bei einer Temperatur im Bereich von 44°C bis 52°C, bevorzugt zwischen 46°C und 50°C und besonders bevorzugt bei 48°C für eine Dauer von 10-40 Minuten, vorzugsweise für 20 Minuten.

Nach erfolgter in situ-Hybridisierung und anschließender Entfernung der nichthybridisierten Nukleinsäuresondenmoleküle mittels des oben beschriebenen Waschschrittes erfolgt die Abtrennung der hybridisierten Sondenmoleküle für die Detektion und, falls erwünscht, Quantifizierung der hybridisierten Sondenmoleküle.

Für diesen Extraktionsschritt sind solche Extraktionsmittel geeignet, die bei einer geeigneten Temperatur eine Denaturierung der Sonde von der Targetsequenz gewährleisten, ohne die Sondenmoleküle in nennenswertem Ausmaß zu beschädigen. Letzteres ist insbesondere deswegen wünschenswert, um die Messergebnisse bei der nachfolgelnden Detektion und Quantifizierung nicht nachteilig zu beeinflussen. Bevorzugt wird hierfür als Ablöselösung bzw. Ablösepuffer Wasser, also $H_2O_{dest./bidest.}$, oder schwach gepuffertes Wasser, also bspw. Tris/HCl, im Konzentrationsbereich von 0,001 M bis 1,0 M, besonders bevorzugt in einer Konzentration von 0,01 M, verwendet, wobei der pH von Tris/HCl zwischen 7,0 und 11,0, vorzugsweise 9,0, beträgt. Weiter sind im Rahmen dieser Erfindung auch DMSO (Dimethylsulfoxid) und 1 x SSC, pH 10,0 (+/- 2,0) als Extraktionsmittel geeignet, wobei 1 x SSC geeigneterweise durch Verdünnung einer 20 x SSC-Stammlösung (175,2 g NaCl, 88,2 g Natriumcitrat, auffüllen mit Wasser auf 1 Liter) zubereitet wird.

Die Ablösung der Sondenmoleküle erfolgt üblicherweise für eine Dauer von 5 bis 30 Minuten, bevorzugt für 15 Minuten. Dabei erfolgt die Sondenextraktion in einem Temperaturbereich von 50-100°C, vorzugsweise bei unter 100°C, insbesondere bevorzugt bei etwa 80°C. In jedem Fall sollte versucht werden, die Extraktion bei einer Temperatur durchzuführen, die eine effektive, aber gleichzeitig für die Sondenmoleküle schonende Abtrennung gewährleistet. Da die Sonden durch die Extraktionsbehandlung umso weniger beeinträchtigt werden, je geringer die Temperatur ist, wird eine Temperatur < 100°C, insbesondere < 90°C bevorzugt.

In einem Vergleichsversuch wurden 4,8 x 10$^6$ *Burkholderia cepacia*-Zellen mit zwei fluoreszenzmarkierten Oligonukleotidsonden (jeweils 2,5 $\mu$l BET42a-Cy3 bzw. NonEUB338-Cy3) mittels des erfindungsgemäßen Fast-FISH-Verfahrens hybridisiert. Die Sondenextraktion erfolgte 15 Minuten bei 80°C mit 110 $\mu$l Ablöselösung, wobei $H_2O_{bidest}$, 0.01 M Tris/HCl, pH 9,0, 1 x SSC, pH 10,0, DMSO und Formamid hinsichtlich des Meßsignals, also der Fluoreszenzintensität miteinander verglichen wurden. Während sowohl mit Wasser, 0.01 M Tris/HCl, 1 x SSC und DMSO gute bis sehr gute Ergebnisse erzielt werden konnte, konnte unter Verwendung von Formamid kein, der Sondenextraktion mit den anderen Ablöselösungen vergleichbares Meßsignal erzielt werden. Vielmehr war das mit Formamid als Extraktionslösung erreichbare Signal um mindestens Faktor 10 geringer als die Intensitäten, die bspw. mit $H_2O_{bidest}$ oder 0.01 M Tris/HCl gemessen werden konnten. Bei einer Ablösetemperatur von 100°C (also einer Temperatur des Extraktionsmittels von 100°C) war der Unterschied in den Meßsignalen zwischen Formamid einerseits und $H_2O_{bidest}$ bzw. 0,01 M Tris/HCl andererseits noch stärker ausgeprägt. Diese Beobachtungen sind höchstwahrscheinlich auf eine noch stärkere Beeinträchtigung der Sondenmoleküle durch Formamid mit zunehmender Temperatur zurückzuführen.

Aufgrund dieser Beobachtungen werden insbesondere $H_2O_{bidest}$ oder 0,01 M Tris/HCl als Extraktionsmittel gegenüber Formamid, das im Stand der Technik in der Regel als Extraktionsmittel empfohlen wird, bevorzugt. Durch den Verzicht von Formamid im Extraktionsschritt erzielt man nicht nur eine schonendere Behandlung, es ergeben sich zusätzlich auch Vorteile hinsichtlich des Gefahrenspotentials, da es sich bei Formamid um eine Substanz der Giftklasse 3 handelt. Darüber hinaus ist die Verwendung von Formamid im Vergleich zu Wasser oder schwach gepuffertem Wasser wesentlich kostenintensiver.

Die Auswahl der jeweiligen Nukleinsäuresonde erfolgt in Abhängigkeit vom nachzuweisenden Mikroorganismus: Sollen beispielsweise nur Mikroorganismen der Gattung *Streptococcus salivarius* nachgewiesen werden, nicht jedoch Mikroorganismen der Gattung *Streptococcus thermophilus,* so wird der Fachmann eine geeignete Sequenz auswählen, die in *Streptococcus salivarius* auftritt, in *Streptococcus thermophilus* dagegen nicht. Typischerweise werden diese Sequenzen aus der 16S-oder 23S-rRNA ausgewählt. Ist dagegen erwünscht, alle Bakterien der Gattung *Streptococcus* zu erfassen, wird eine Sequenz ausgewählt werden, die *Streptococcus salivarius* und *Streptococcus thermophilus* sowie weiterer Spezies der Gattung *Streptococcus* gemeinsam ist. Für solche Sequenzen sind in der Literatur bereits viele Beispiele veröffentlicht, siehe z.B. Beimfohr et al. (1993) System Appl. Microbiol. 16:450. Die Nukleinsäuresonde kann dabei komplementär zu einer chromosomalen oder episomalen DNA sein, aber auch zu einer mRNA oder rRNA des nachzuweisenden Mikroorganismus. Dabei ist es bevorzugt, Nukleinsäuresonden zu wählen, die einem Bereich komplementär sind, der in einer Kopienzahl von mehr als 1 im jeweiligen nachzuweisenden Mikroorganismus vorliegt. Die nachzuweisende Sequenz liegt bevorzugt 500 - 100.000 mal pro Zelle vor, besonders bevorzugt 1000 - 50.000 mal. Dies Ist ein weiterer Grund, warum die Nukleinsäuresonde besonders bevorzugt komplementär zu einer rRNA ist: die ribosomalen RNAs sind Bestandteile der Ribosomen, die, da sie die Proteinsynthesemoleküle darstellen, in jeder aktiven Zelle vieltausendfach vorhanden sind.

Erfindungsgemäß wird die Nukleinsäuresonde mit dem im obengenannten Sinne fixierten Mikroorganismus inkubiert, um so ein Eindringen der Nukleinsäuresondenmoleküle in den Mikroorganismus und die Hybridisierung von Nukleinsäuresondenmolekülen mit den Nukleinsäuren des Mikroorganismus zu erlauben. Daran anschließend werden nicht hybridisierte Nukleinsäuresondenmoleküle durch übliche Waschschritte entfernt. Im Gegensatz zum herkömmlichen F1SH-Verfahren werden nunmehr jedoch nicht die hybridisierten Nukleinsäuresondenmoleküle in situ, also im jeweiligen Mikroorganismus, belassen, sondern von der nachzuweisenden Nukleinsäure wiederum abgelöst und von zellulären Bestandteilen getrennt, detektiert und gegebenenfalls quantifiziert. Voraussetzung dafür ist, daß das erfindungsgemäß verwendete Nukleinsäuresondenmolekül nachweisbar ist. Diese Nachweisbarkeit kann z.B. durch kovalente Verbindung des Nukleinsäuresondenmoleküls mit einem detektierbaren Marker sichergestellt werden. Als detektierbare Marker werden üblicherweise fluoreszierende Gruppen, z.B. Cy-2, Cy-3 oder Cy-5, FITC, CT, TRITC oder Fluos-Prime verwendet, die dem Fachmann alle wohlbekannt sind; der Vollständigkeit halber sind einige Marker, ihre Eigenschaften und Bezugsquellen in der folgenden Tabelle 1 angegeben.

TABELLE 1

FLUOS: 5,(6)-Carboxyfluorescein-N-hydroxysuccinimidester (Boehringer Mannheim, Mannheim, Deutschland); $\varepsilon = 7{,}50 \times 10^4$ mol$^{-1}$ l$^{-1}$, $Abs_{max}$ bei 494 nm; $Em_{max}$ bei 518 nm, MG = 473.

TRITC: Tetramethylrhodamin-5,6-isothiocyanat (Isomer G. Molecular Probes Inc.,Eugene, USA, Lambda, Graz. AT); $\varepsilon = 1{,}07 \times 10^5$ mol$^{-1}$ 1$^{-1}$, $Abs_{max}$ bei 537 nm; $Em_{max}$ bei 566 nm, MG = 479.

CT: 5,(6)-Carboxytetramethylrhodamin-N-hydroxysuccinimidester (Molecular Probes Inc., Eugene, USA); $\varepsilon = 0{,}87 \times 10^5$ mol$^{-1}$ l$^{-1}$, $Abs_{max}$ bei 537 nm; $Em_{max}$ bei 566 nm.

CY-3: NHS-Ester von Cy5.18 (Biological Detection Systems, Pittsburgh. USA); (Amersham Life Sciences, Inc., Arlington Heights, USA); $\varepsilon = 1{,}5 \times 10^5$ mol$^{-1}$ l$^{-1}$, $Abs_{max}$ bei 532 nm; $Em_{max}$ bei 565 nm. MG = 765,95.

CY-5: NHS-Ester von Cy5.18 (Biological Detection Systems, Pittsburgh, USA); (Amersham Life Sciences, Inc., Arlington Heights, USA); $\varepsilon = > 2 \times 10^5$ mol$^{-1}$ 1$^{-1}$, $Abs_{max}$ bei 650 nm; $Em_{max}$ bei 667 nm. MG = 791,99.

Bei Verwendung von fluoreszierenden Markern wird das erfindungsgemäße Verfahren in Anlehnung an das oben erwähnte "FISH"-Verfahren im folgenden auch als "Fast-FISH"-Verfahren bezeichnet.

Alternativ werden chemolumineszierende Gruppen oder radioaktive Markierungen, z.B. $^{35}$S, $^{32}$P, $^{33}$P, $^{125}$J, verwendet. Nachweisbarkeit kann aber auch gegeben sein durch Kopplung des Nukleinsäuresondenmoleküls mit einem enzymatisch aktiven Molekül, beispielsweise alkalischer Phosphatase, saurer Phosphatase, Peroxidase, Meerrettichperoxidase, β-D-Galaktosidase oder Glukoseoxidase. Für jedes dieser Enzyme ist eine Reihe von Chromogenen bekannt, die anstelle des natürlichen Substrats umgesetzt werden können, und entweder zu farbigen oder zu fluoreszierenden Produkten umgesetzt werden können. Beispiele für solche Chromogene sind in der nachfolgenden Tabelle 2 angegeben.

TABELLE 2

| Enzyme | Chromogen |
| --- | --- |
| 1. Alkalische Phosphatase und saure Phosphatase | 4-Methylumbelliferylphosphat (*), |
| | Bis(4-Methyiumbelliferylphosphat), |
| | (*) 3-O- Methylfluoreszein, |
| | Flavon-3-Diphosphattriammonium-salz (*), |
| | p-Nitrophenylphosphatdinatriumsalz |
| 2. Peroxidase | Tyraminhydrochlorid (*), |
| | 3-(p-Hydroxyphenyl)-Propionsäure (*), |
| | p-Hydroxyphenethylalkohol (*), |
| | 2,2'-Azino-di-3-ethylbenzthiazolinsulfonsäure (ABTS), |
| | ortho-Phenylendiamindihydrochlorid, |
| | o-Dianisidin, 5-Aminosalicylsäure, |
| | p-Ucresol (*), |
| | 3,3'-Dimethyloxybenzidin, |
| | 3-Methyl-2-benzothiazolin-hydrazon, |
| | Tetramethylbenzidin |
| 3. Meerrettichperoxidase | $H_2O_2$ + Diammoniumbenzidin |
| | $H_2O_2$ + Tetramethylbenzidin |
| 4. β-D-Galaktosidase | o-Nitrophenyl-β-D-galaktopyranosid, |
| | 4-Methylumbelliferyl-β-D-galaktosid |
| 5. Glukoseoxidase | ABTS, |
| | Glukose und Thiazolylblau |
| * Fluoreszenz | |

Schließlich ist es möglich, die Nukleinsäuresondenmoleküle so zu gestalten, daß an ihrem 5'- oder 3'-Ende eine weitere zur Hybridisierung geeignete Nukleinsäuresequenz vorhanden ist. Diese Nukleinsäuresequenz umfaßt wiederum ca. 15 bis 1000, bevorzugt 15 bis 50 Nukleotide. Dieser zweite Nukleinsäurebereich kann wiederum von Oligonukleotidsonden erkannt werden, die durch eines der obenerwähnten Mittel nachweisbar sind.

Eine weitere Möglichkeit besteht in der Kopplung der nachweisbaren Nukleinsäuresondenmoleküle mit einem Hapten. Nach Ablösung der Nukleinsäuresondenmoleküle von der Zielnukleinsäure können die nunmehr separat vorliegenden Nukleinsäuresondenmoleküle mit das Hapten erkennenden nachweisbaren Antikörpern in Kontakt gebracht werden. Ein bekanntes Beispiel für ein solches Hapten ist Digoxigenin oder seine Derivate. Dem Fachmann sind über die angegebenen Beispiele hinaus viele weitere Möglichkeiten bekannt, ein zur Hybridisierung verwendetes Oligonukleotid zu detektieren und zu quantifizieren.

Die Vielzahl möglicher Markierungen ermöglicht auch den gleichzeitigen Nachweis von zwei oder mehr verschiedenen, sich überlappenden oder sich nicht überlappenden Populationen. So kann z.B. durch Verwendung von 2 verschiedenen Fluoreszenzmarkern *Streptococcus salivarius* neben *Streptococcus thermophilus,* oder *Streptococcus salivarius* neben der Streptococcen-Gesamtpopulation nachgewiesen werden.

Der mittels des erfindungsgemäßen Verfahrens nachzuweisende Mikroorganismus kann ein prokaryontischer oder eukaryontischer Mikroorganismus sein. In den meisten Fällen wird es erwünscht sein, einzellige Mikroorganismen nachzuweisen. Relevante Mikroorganismen sind dabei vor allem Hefen, Bakterien, Algen oder Pilze.

In einer besonders bevorzugten Ausführungsform der vorliegenden Erfindung handelt es sich bei dem Mikroorganismus um einen Angehörigen der Gattung *Salmonella.*

Das erfindungsgemäße Verfahren kann vielfältig angewendet werden. So ist es z.B. geeignet, Umweltproben auf das Vorhandensein bestimmter Mikroorganismen zu untersuchen. Diese Umweltproben können aus dem Wasser, aus dem Boden oder aus der Luft entnommen sein. Für den Nachweis von bestimmten Bakterien in Umweltproben ist normalerweise keinerlei vorausgehende Kultivierung notwendig.

Ein weiteres wichtiges Anwendungsgebiet für das erfindungsgemäße Verfahren ist die Kontrolle von Lebensmittelproben. In bevorzugten Ausführungsformen werden die Lebensmittelproben aus Milch oder Milchprodukten (Joghurt, Quark, Käse, Butter, Buttermilch), Trinkwasser, Getränken (Säfte, Limonade, Bier), Backwaren oder Fleischwaren entnommen. Für den Nachweis von Mikroorganismen in Lebensmitteln kann u. U. eine vorherige Kultivierung erwünscht oder sogar vorgeschrieben sein. So ist es notwendig, z. B. für den Nachweis von einer einzigen Salmonelle in 25 ml Milch, diese eine zeitlang zu kultivieren, um anschließend auch mit statistischer Zuverlässigkeit eine oder mehrere Salmonellen im Probenvolumen zu haben.

Das erfindungsgemäße Verfahren kann weiter zur Untersuchung medizinischer Proben eingesetzt werden. Dabei ist es sowohl für die Untersuchung von Gewebeproben, z.B. Biopsiematerial aus der Lunge, Tumor- oder entzündlichem Gewebe, aus Sekreten wie Schweiß, Speichel, Sperma und Ausfluß aus der Nase, Harnröhre oder Vagina sowie für Stuhl- und Urinuntersuchungen geeignet.

Ein weiteres Anwendungsgebiet für das vorliegende Verfahren ist die Untersuchung von Ahwässern, z.B. Belebtschlamm, Faulschlamm oder anaerobem Schlamm. Darüber hinaus ist es geeignet, Biofilme in industriellen Anlagen zu analysieren, sowie auch sich natürlicherweise bildende Biofilme oder bei der Abwasserreinigung bildende Biofilme zu untersuchen. Schließlich ist es auch zur Untersuchung und Qualitätskontrolle pharmazeutischer und kosmetischer Produkte, z.B. von Salben, Cremes. Tinkturen, Säften etc. geeignet.

Das erfindungsgemäße Verfahren stellt eine Möglichkeit dar, die in situ-Hybridisierung zur Zellidentifizierung und gegebenenfalls Quantifizierung in der Praxis zu etablieren. Die erforderliche Ausstattung würde sich z.B. bei Verwendung von Fluoreszenzmolekülsonden auf den Erwerb eines Fluorometers beschränken (max. ca. DM 18000,--). Im Gegensatz dazu bewegt sich ein Epifluoreszenzmikroskop, das zur Durchführung des herkömmlichen FISH-Verfahrens geeignet ist, und mit welchem ausreichend gute in situ-Hybridisierungsergebnisse erzielt werden können, in der Preisklasse von ca. DM 100000.--. Hinzu kommt, daß bei Verwendung z. B. von Cy-5 markierten Sonden, die Epifluoreszenzmikroskope zusätzlich mit einer hochwertigen CCD-Kamera ausgestattet sein müssen (Preis zwischen DM 30000,-- und DM 50000,--). Aus diesem Grund stellt das erfindungsgemäße Verfahren eine wesentlich billigere Meßmethode dar, als die zeitaufwendige Quantifizierung am Epifluoreszenzmikroskop. Überdies sind die laufenden Kosten des erfindungsgemäßen Verfahrens mittels Fluorometer wesentlich geringer als die des herkömmlichen Verfahrens mittels Epifluoreszenzmikroskopie. Dies liegt vor allem daran, daß die Quecksilberhochdrucklampen (DM 450,-- pro Stück) der Epifluoreszenzmikroskope aus Qualitäts- und Sicherheitsgründen spätestens alle 100 Betriebsstunden erneuert werden müssen. Das enorm zeitaufwendige Zählen spezifisch markierter Zellen unter dem Mikroskop führt somit zu einem hohen Lampenverschleiß. Die Xenonbogenlampe eines Fluorometers (DM 3000,--) hat selbst bei intensiver Auslastung des Gerätes eine Haltbarkeit von 1 bis 3 Jahren. Einen zusätzlichen Kostenfaktor stellen auch die für die Messung benötigten Personalkosten dar. Während die quantitative Analyse einer Umweltprobe mittels des herkömmlichen Verfahrens vor allem beim Einsatz mehrerer Sonden mehrere Tage in Anspruch nimmt, sollte das erfindungsgemäße Verfahren diese Aufgabe innerhalb weniger Stunden erledigen. Für die Hybridisierung und Extraktion wird ein Zeitaufwand von 3 Stunden benötigt, die Quantifizierung im Fluorometer wird nur wenige Minuten in Anspruch nehmen. Die Quantifizierung könnte auch von ungeschulten Kräften vorgenommen werden, wohingegen bei dem herkömmlichen Verfahren die visuelle Quantifizierung die Fähigkeiten eines Spezialisten erfordert.

Damit wird erfindungsgemäß ein Verfahren bereitgestellt, das im Vergleich mit den herkömmlichen Verfahren umweltfreundlicher sowie schneller und leichter durchzuführen ist.

Obwohl die Erfindung im wesentlichen mit Bezug auf fluoreszenzmarkierte Sondenmoleküle beschrieben worden ist, versteht es sich von selbst, daß die genannten Vorteile bei Verwendung anderer Marker ebenfalls gegeben sind.

Erfindungsgemäß wird weiterhin ein Kit zur Durchführung des Verfahrens zum Nachweisen von Mikroorganismen in einer Probe bereitgestellt. Der Inhalt eines solchen Kits richtet sich im wesentlichen nach der Natur des nachzuweisenden Mikroorganismus. Er umfaßt als wichtigsten Bestandteil eine für den jeweils nachzuweisenden Mikroorganismus spezifische Nukleinsäuresonde sowie eine weitere Nukleinsäuresonde, mit der eine Negativkontrolle durchgeführt werden kann. Darüber hinaus umfaßt er einen Hybridisierungspuffer und gegebenenfalls einen Lysepuffer. Die Wahl des Hybridisierungspuffers hängt in erster Linie von der Länge der verwendeten Nukleinsäuresonden ab. So müssen, wie dem Fachmann bekannt ist, für die Hybridisierung einer Nukleinsäuresonde von 15 Nukleotiden Länge weniger stringente Bedingung gewählt werden als für die Hybridisierung einer Sonde von 75 Nukleotiden Länge. Beispiele für Hybridisierungsbedingungen sind z. B. in Stahl & Amann (1991) in Stackebrandt u. Goodfellow (Hrsg.), Nucleic Acid Techniques in Bacterial Systematics; John Wiley & Sons Ltd., Chichester, UK, angegeben.

Die Zusammensetzung des Lysepuffers ist ebenfalls vom jeweiligen Mikroorganismus abhängig. So sind zur Lyse von Virushüllen, Zellwänden Grampositiver oder Gram-negativer Bakterien, Zellmembranen von Hefe oder Algen jeweils leicht unterschiedliche Bedingungen erforderlich, die ohne weiteres in der Literatur festgestellt werden können.

In einer bevorzugten Ausführungsform enthält der erfindungsgemäße Kit spezifische Sonden zum Nachweis von Bakterien der Gattung *Salmonella*. In einer besonders bevorzugten Ausführungsform handelt es sich bei dem Nukleinsäuresondenmolekül zum spezifischen Nachweis eines Mikroorganismus um die Nukleinsäuresequenz
Salm63: 5'-TCGACTGACTTCAGCTCC-3' und bei der Negativkontrolle um die Sequenz
NonSalm: 5'-GCTAACTACTTCTGGAGC-3' oder um Nukleinsäuresondenmoleküle, die sich von Salm63 und/oder NonSalm durch eine Deletion und/oder Addition unterscheiden, wobei die Fähigkeit dieser Sonden, mit *Salmonella*-spezifischer Nukleinsäure zu hybridisieren, erhalten bleibt, oder um Nukleinsäuresondenmoleküle, die mit den zuvor genannten Nukleinsäuren hybridisieren können.

Die folgenden Abbildungen und die Beispiele dienen der Erläuterung der Erfindung und sollen nicht in einschränkender Weise ausgelegt werden.

Abbildungen

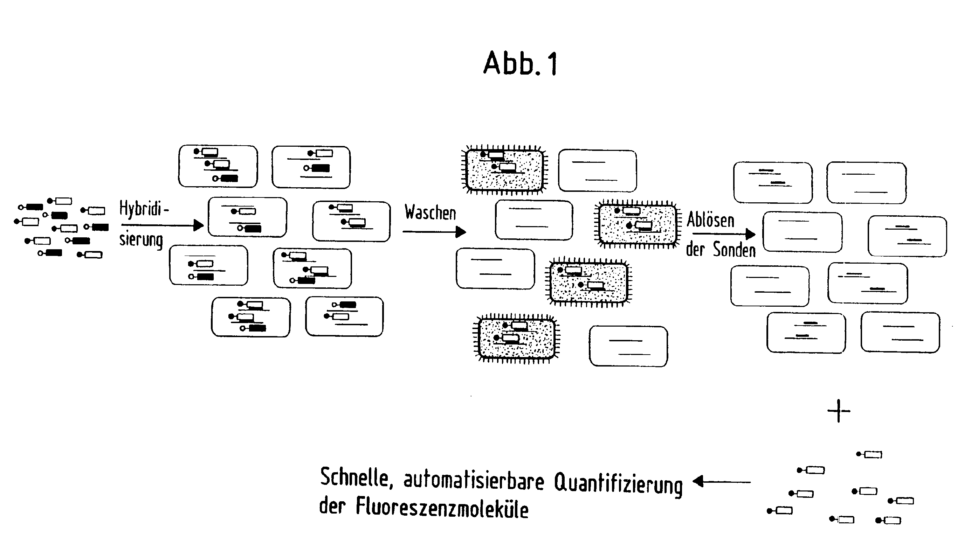

Abbildung 1 veranschaulicht, wie das erfindungsgemäße Verfahren zur Detektion von Zellen eines Typs A eingesetzt werden kann, während gleichzeitig die Anwesenheit von Zellen eines Typs B ausgeschlossen wird. Dazu werden Nukleinsäuresonden, die für die Zelltypen A und B spezifisch sind, bereitgestellt und mit der zu untersuchenden Probe hybridisiert. Während des Hybridisierungsvorganges dringen die verschiedenartig markierten Sonden A und B in die Zellen vom Typ A und C ein. Nur die Zelle vom Typ A enthält Zielnukleinsäure mit den Bindungsstellen für Sonden vom Typ A. nicht jedoch die Zelle C. Darüber hinaus besitzt keine der beiden Zellen Bindungsstellen für Sonden vom Typ B. der deshalb nicht gebunden wird. Nach dem anschließenden Waschschritt befinden sich nur noch gebundene Nukleinsäuresonden vom Typ A in den Zellen.

Die Mischung von Zellen, die zum Teil Nukleinsäuresondenmoleküle vom Typ A gebunden haben, wird einem Ablöseschritt unterworfen. Anschließend können die nun abgelösten Nukleinsäuresondenmoleküle vom Typ A quantifiziert werden.

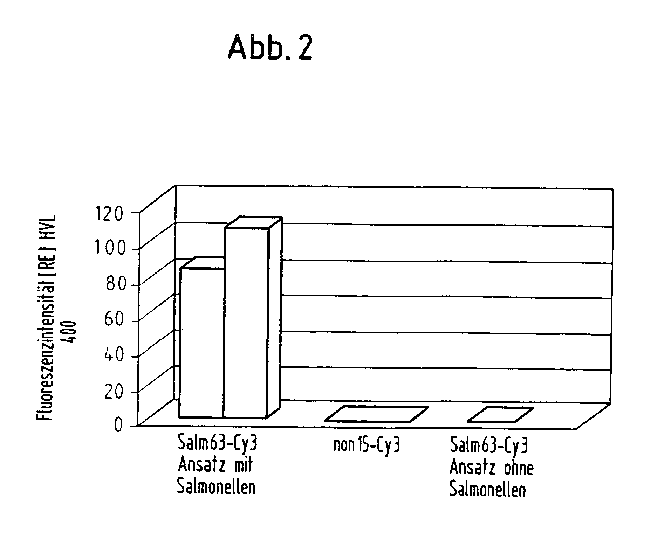

Abbildung 2 zeigt das Ergebnis einer Hybridisierung von 10 in H-Milch inokulierten *S. typhimurium* LT2 Zellen nach 13stündiger Inkubation bei 37°C, 40 % im HP, HVL 400.

Ansatz mit Salmonellen:

Salm63-Cy3       erfaßt *Salmonella* spec., stellt spezifisches Signal dar

non15-Cy3        Nonsense-Sonde, repräsentiert unspezifische Bindung und Hintergrund
                 (Kontrolle 1)

Ansatz ohne Salmonellen:

Salm63-Cy3 erfaßt *Salmonella* spec, repräsentiert den Hintergrund mit der Salmonellen-spezifischen Sonde, da in diesem Ansatz keine Salmonellen vorhanden waren (Kontrolle 2)

BEISPIEL 1

Nachweis von Bakterien der Gattung *Salmonella* in Milch

1. Allgemeine Beschreibung:

Das nachfolgend beschriebene Verfahren, im Rahmen dieser Erfindung "SalmoQuick-Verfahren" genannt, dient zur qualitativen Analyse von Bakterien der Gattung *Salmonella* in Lebensmitteln auf der Grundlage des erfindungsgemäßen Verfahrens. Die Identifizierung von Salmonellen erfolgt in 24 Stunden; dadurch ergibt sich ein erheblicher Geschwindigkeitsvorteil gegenüber konventionellen Methoden, die für eine Identifizierung je nach taxonomischer Genauigkeit zwischen 5 und 14 Tagen benötigen.

2. Grundprinzip:

Salmonellen in Milch werden durch gegen rRNA-gerichtete fluoreszenzmarkierte Oligonukleotidsonden spezifisch erfaßt. Nach entsprechend stringenten Waschschritten werden die gebundenen Sonden wieder von ihren Zielstellen in den Bakterien abgelöst und in einem Fluorometer quantifiziert. Durch die Höhe des erhaltenen Signals im Fluorometer kann eine Aussage darüber getroffen werden, ob in der Milch Salmonellen anwesend sind oder nicht.

3. Kurzbeschreibung:

Die Milchprobe, die auf die Anwesenheit von Salmonellen untersucht werden soll, wird mehrere Stunden inkubiert. Auf diese Weise wird sichergestellt, daß durch die Vermehrung der eventuell in der Milch vorhandenen Salmonellen erstens genügend Zielstellen für die Detektion mit Sonden vorhanden sind und zweitens nur lebende Salmonellen identifiziert werden. Ein Populationsshift durch die mehrstündige Inkubation ist unschädlich, da es nur um die Gegenwart oder Abwesenheit von Salmonellen' nicht jedoch um den Nachweis aller Bakterien geht. Nach der Zentrifugation und der Fixierung der Zellen, während der die Zellen für die Sonden zugänglich gemacht werden, können durch einen Lyseschritt die die anschließende Hybridisierung störenden Proteine ausreichend gut entfernt werden. Während der anschließenden Hybridisierung binden unter ausreichend stringenten Bedingungen die fluoreszenzmarkierten Oligonukleotidsonden spezifisch an die rRNA oder Bakterien der Gattung *Salmonella.* Der anschließende Waschschritt sorgt für eine Entfernung der nicht gebundenen Sonden. Während einer weiteren Behandlungsprozedur werden die spezifisch gebundenen Sonden aus den Zellen extrahiert. Die Fluoreszenzfarbstoffe dieser Sonden können dann in einem Fluorometer quantifiziert werden. Die Höhe des erhaltenen Signals gibt Auskunft darüber, ob Salmonellen in der Milchprobe vorhanden waren oder nicht.

4. Technische Ausstattung:

4.1 Vorbereitung der Probe
Zur Probenvorbereitung werden benötigt:

- Probengefäß (Greiner, Nürtingen)
- Rundschüttler
- Greiner-Zentrifuge (8000 Umdrehungen / Minute)
- Tischzentrifuge (14000 Umdrehungen / Minute)

4.2 In situ-Hybridisierung
Für die in situ-Hybridisierung werden benötigt:

- Tischzentrifuge (14000 Umdrehungen / Minute)
- Heizblock und Wasserbad oder zwei Heizblöcke
- Hybridisierungsofen

4.3 Quantifizierungsgerät

- Fluorometer, Chemiluminometer oder Szintillator

5. Materialien

5.1 Vorbereitung der Probe
Folgende Materialien wurden zur Verarbeitung der Probe einschließlich der Zellfixierung verwendet:
• 9 ml Saline-Röhrchen 0,9 % NaCl in $H_2O_{dest.}$
• Tryptic Soy Medium (TS-Medium)

| | | |
|---|---|---|
| Casein Pepton | 15,0 g | |
| Soja Pepton | 5,0 g | |
| NaCl | 5,0 g | |
| $H_2O_{dest.}$ | ad 1 l | pH 7,3 |

• Lyse-Puffer:

| | |
|---|---|
| $Na_2HPO_4$ | 100 mM |
| NaCl | 150 mM |
| EDTA | 10 mM |
| NaOH | 40 mM |

• PBS-Stammlösung ($Na_xPO_4$)

| | |
|---|---|
| 200 mM $NaH_2PO_4$ | |
| 200 mM $Na_2HPO_4$ | pH 7,2 - 7,4 |

• 3 x PBS-Lösung

390 mM NaCl
30 mM $Na_xPO_4$ (PBS-Stammlösung)     pH 7,2 - 7,4

• 1 x PBS-Lösung

130 mM NaCl
10 mM $Na_xPO_4$ (PBS-Stammlösung)     pH 7,2 - 7,4

• 4% Paraformaldehydlösung (PFA):

Herstellbar durch Zugabe von 3 g Paraformaldehyd zu 30 ml auf 60°C erhitztem $H_2O_{bidest.}$; tropfenweise Zugabe von 1 M NaOH bis zum vollständigen Lösen des Paraformaldehyds; anschließend Hinzufügen von 16,6 ml 3 x PBS, Abkühlung der Lösung auf ca. 20°C; Einstellen des pH-Wertes mit 1 M HCl auf 7,2 - 7,4; Sterilfiltration der fertigen PFA-Lösung über einen 0,2$\mu$m-Filter (MILLIPORE, Eschborn). Die Lösung kann bei 4°C für ca. eine Woche aufbewahrt werden; Einfrieren über mehrere Monate ist ebenfalls möglich.

5.2 In situ-Hybridisierung

• Formamid (Merk, Darmstadt)
• Sonden-Arbeitslösungen zu je 50 ng/$\mu$l (spezifische und unspezifische Sonde)

Ablösepuffer (2 ml):

0,01 M Tris/HCl     pH 9,0

Hybridisierungspuffer (HP, 2 ml mit x% Formamidgehalt):

| | | |
|---|---|---|
| 5 M NaCl | | 360 $\mu$l |
| 1 M Tris/HCl | pH 8,0 | 40 $\mu$l |
| Formamid | | y $\mu$l |
| 10% (w/v) SDS | | 2 $\mu$l |
| ad $H_2O_{bidest.}$ | | 2 ml |

Waschpuffer (2 ml):

| | | |
|---|---|---|
| 5 M NaCl | | 18,4 $\mu$l |
| 1 M Tris/HCl | pH 8,0 | 40 $\mu$l |
| 0.5 M EDTA | pH 8,0 | 20 $\mu$l |
| 10% (w/v) SDS | | 2 $\mu$l |
| ad $H_2O_{bidest.}$ | | 2 ml |

Sonden:

Salm63: 5'-TCGACTGACTTCAGCTCC-3'
NonSalm: 5'-GCTAACTACTTCTGGAGC-3'

verwendeter Fluoreszenzfarbstoff: Cy3

6. Durchführung

6.1 Vorbereitung der Milch

1. Milchproben und TS-Medium ca. 20 min auf 37°C vorwärmen.

2. Zugabe von 25 ml vorgewärmtem TS-Medium zu 25 ml vorgewärmter Milch in einem 50 ml fassenden Probengefäß.

3. Inkubation bei 30° über Nacht (13 Stunden) auf dem Rundschüttler.

4. Zentrifugation der 50 ml Probengefäße zur Zellernte bei 8000 Umdrehungen/min für 8 min.

5. Resuspension des Pellets mit 25 ml Lysepuffer zur Trennung von Proteinen und Fetten der Milch von den Zellen und anschließende Inkubation für 10 min bei RT (Raumtemperatur).

6. Zentrifugation für 8 min bei 8000 Umdrehungen/min.

7. Wiederholung der Resuspension und Inkubation mit Lysepuffer (siehe 5.)

8. Zentrifugation für 8 min bei 8000 Umdrehungen/min.

9. Resuspension der pelletierten Zellen in 600 $\mu$l 1 x PBS und Überführung in ein Eppendorf-Reaktionsgefäß. Spülen des 50 ml Probengefäßes mit 600 $\mu$l 1 x PBS.

10. Zentrifugation der Eppendorf-Reaktionsgefäße bei 14000 Umdrehungen/min für 6 min.

11. Verwerfen des Überstandes und Resuspension der Zellen in 1 ml 1 x PBS.

12. Zentrifugation der Eppendorf-Reaktionsgefäße bei 14000 Umdrehungen/min für 6 min.

13. Verwerfen des Überstandes und vollständige Resuspension des Pellets in 200 $\mu$l 1 x PBS.

14. Zugabe von 600 $\mu$l einer frisch hergestellten 4%igen Paraformaldehydlösung.

15. Inkubation für 1 Stunde bei 4°C.

16. Zentrifugation bei 14000 Umdrehungen/min für 6 min.

17. Verwerfen des Überstandes und vollständige Resuspension des Pellets in 1 ml 1 x PBS.

18. Zentrifugation bei 14000 Umdrehungen/min für 6 min.

19. Verwerfen des Überstandes (Pellet verbleibt im Cap).

6.2 In situ-Hybridisierung

1. Vorwärmen des Hybridisierungspuffers auf 48°C im Wasserbad oder im Heizblock für ca. 20 min.

2. Überführung der Eppendorf-Reaktionsgefäße (mit dem zurückgebliebenen Zellpellet) auf den Heizblock (auf 80°C vorgeheizt) und Inkubation für 5 min bei 80°C.

3. Nach der 5 minütigen Inkubation bei 80°C Zugabe von 80 µl des vorgewärmten Hybridisierungspuffers in jedes Eppendorf-Reaktionsgefäß.

4. Zugabe von je 2,5 µl der frisch aufgetauten Sonden-Arbeitslösung "Salm63-Cy3" zu zwei der vier Milchproben-Ansätze.

5. Zugabe von je 2,5 µl der frisch aufgetauten Sonden-Arbeitslösung "nonSalm63-Cy3" zu den beiden verbleibenden Milchproben-Ansätzen.

6. Heftig Durchmischen der Eppendorf-Reaktionsgefäße für 10 Sekunden und kurzes (2 Sekunden) Abzentrifugieren mittels einer kleinen Tischzentrifuge.

7. Inkubation der Eppendorf-Reaktionsgefäße für 2 Stunden bei 46°C im Hybridisierungsofen oder im Wasserbad bzw. Heizblock (Hybridisierung der Sonde mit der Zielsequenz unter stringenten Bedingungen).

8. Vorwärmen des Waschpuffers auf 48°C im Wasserbad oder im Heizblock.

9. Nach Inkubation für 2 Stunden bei 46°C Zentrifugation der Eppendorf-Reaktionsgefäße bei 14000 Umdrehungen/min für 6 min.

10. Vollständiges Abnehmen und Verwerfen des Überstandes mit einer 200 µl-Pipette ohne das Pellet zu berühren.

11. Zugabe von 100 µl vorgewärmtem Waschpuffer, heftiges Durchmischen der Eppendorf-Reaktionsgefäße für 5 Sekunden und Überführung ins Wasserbad bei 48°C für 20 Minuten (Entfernen der nicht gebundenen Sondenmoleküle unter stringenten Bedingungen).

12. Vorwärmen des Ablösepuffers auf 80°C im Heizblock.

13. Nach 20 minütiger Inkubation bei 48°C Zentrifugation der Eppendorf-Reaktionsgefäße bei 14000 Umdrehungen/min. für 6 min.

14. Vollständiges Abnehmen und Verwerfen des Überstandes mit einer 200 µl-Pipette ohne das Pellet zu berühren.

15. Zugabe von 110 µl vorgewärmtem Ablösepuffer, heftiges Durchmischen der Eppendorf-Reaktionsgefäße für 5 Sekunden und Überführung auf den Heizblock auf 80°C für 15 min (Ablösung der Sonde von der Zielsequenz).

16. Nach 15 minütiger Inkubation bei 80°C Zentrifugation der Eppendorf-Reaktionsgefäße bei 14000 Umdrehungen/min für 6 min.

17. Vorsichtige Überführung des Überstandes mit einer 200 µl-Pipette ohne das Pellet zu berühren in neue 1.5 ml Reaktionsgefäße und anschließend Lagerung auf Eis im Dunkeln bis zur Vermessung.

18. Fluorometer anschalten und die Wellenlängen auf 550 nm zur Anregung ("Excitation wavelength") und auf 570 nm ("Emission wavelength") zur Messung der Emission des Cy3-Farbstoffes einstellen.

19. High Voltage Level auf die gewünschte Empfindlichkeit einstellen (400 bis 800 HVL).

20. 107 µl Ablösepuffer in eine Präzisionsglasküvette für die Fluorometrie füllen.

21. Fluorometer auf Null stellen ("Autozero").

22. Eppendorf Reaktionsgefäße kurz vor der Vermessung 5 Sekunden in der Hand auf Raumtemperatur vorwärmen.

23. Einfüllen des die abgelöste Sonde enthaltenden Ablösepuffers in die Präzisionsglasküvette für die Fluorometrie und Vermessung des Signals.

24. Ablesen des Signals nach 10 Sekunden, da nach dem Öffnen der Abdeckung des Lichtkanals das Signal nach 5 bis 8 Sekunden stabil ist.

25. Fluoreszenzwert der untersuchten Milchprobe = Fluoreszenzwert der mit "Salm63-Cy3" hybridisierten Milchprobe abzüglich dem Fluoreszenzwert der mit "nonSalm-Cy3" hybridisierten Milchprobe.

7. Ergebnis

Das Ergebnis des oben beschriebenen Versuches ist in Abb. 2 gezeigt. Es ist eindeutig zu sehen, daß das Salmonellen-spezifische Signal um ein Vielfaches höher ist als eine ebenfalls geprüfte unspezifische Bindung. Auch der Sondenhintergrund war kaum nachweisbar.

BEISPIEL 2

Nachweis von Bakterien der Gattung *Salmonella* in 25 ml Milch bzw. 25 g Milchpulver
Ansatz von zwei Milchproben von je 25 ml bzw. 25 g zur Hybridisierung mit den Sonden "Salm63-Cy3" (Cy3-5'-TCGACTGACTTCAGCTCC-3') und "nonSalm-Cy3" (Cy3-5'-GCTAACTACTTCTGGAGC-3').

A. Zellfixierung:

Laborausstattung:

- 100 ml- und 1 1-Erlemneyerkolben
- Rundschüttler
- Probengefäß (Greiner, Nürtingen)
- Greiner Zentrifuge (8000 Umdrehungen / Minute)
- Tischzentrifuge (14000 Umdrehungen / Minute)

Materialien:

• 9 ml Saline-Röhrchen 0,9 % NaCl in $H_2O_{dest}$
• gepuffertes Peptonwasser (nach §35 LMBG) mit Malachitgrünzusatz

| | |
|---|---|
| Fleisch Pepton | 10,0 g |
| NaCl | 5,0 g |
| Dinatriumhydrogenphosphat (*12 $H_2O$) | 9,0 g |
| Kaliumdihydrogenphosphat | 1,5 g |
| Malachitgrün (Oxalat) | 0,1 g |
| $H_2O_{dest}$ | ad 1 l    pH 7,2 +/- 0,2 |

225 ml-Portionen in 1 1-Erlenmeyerkolben und 30 ml-Portionen in 100 ml-Erlenmeyerkolben abfüllen und 15 min bei 121°C autoklavieren.
• Lyse-Puffer:

| | |
|---|---|
| $Na_2HPO_4$ | 100 mM |
| NaCl | 150 mM |
| EDTA | 10 mM |
| NaOH | 40 mM |

Zur PFA-Zellfixierung verwendete Lösungen:
• PBS-Stammlösung ($Na_xPO_4$)

$$200 \text{ mM NaH}_2\text{PO}_4$$
$$200 \text{ mM Na}_2\text{HPO}_4 \quad \text{pH 7,2 - 7,4}$$

• 3 x PBS-Lösung

$$390 \text{ mM NaCl}$$
$$30 \text{ mM Na}_x\text{PO}_4 \text{ (PBS-Stammlösung)} \quad \text{pH 7,2 - 7,4}$$

• 1 x PBS-Lösung

$$130 \text{ mM NaCl}$$
$$10 \text{ mM Na}_x\text{PO}_4 \text{ (PBS-Stammlösung)} \quad \text{pH 7,2 - 7,4}$$

• 4% Paraformaldehydlösung (PFA):
Herstellbar durch Zugabe von 3 g Paraformaldehyd zu 30 ml auf 60°C erhitztem $H_2O_{bidest}$; tropfenweise Zugabe von 1 M NaOH bis zum vollständigen Lösen des Paraformaldehyds; anschließend Hinzufügen von 16,6 ml 3 x PBS, Abkühlung der Lösung auf ca. 20°C; Einstellen des pH-Wertes mit 1 M HCl auf 7,2 - 7,4; Sterilfiltration der fertigen PFA-Lösung über einen 0,2$\mu$m-Filter (MILLIPORE, Eschborn). Die Lösung kann bei 4°C für ca. eine Woche aufbewahrt werden; Einfrieren über mehrere Monate ist ebenfalls möglich.

Durchführung:

1. 225 ml gepuffertes Peptonwasser mit Malachitgrünzusatz auf 37 °C ca. 1 Stunde vorwärmen.

2. Zugabe von 25 ml Milch bzw. 25 g Milchpulver zu 225ml vorgewärmtem gepufferten Peptonwasser mit Malachitgrünzusatz und gründlich mischen.

3. Inkubation für 7,5 Stunden bei 37°C auf dem Rundschüttler.

4. Sterile Entnahme von 1 ml aus inkubiertem Ansatz und Überführung in 30 ml gepuffertes Peptonwasser mit Malachitgrünzusatz.

5. Inkubation über Nacht (mind. 14 Stunden) bei 37°C auf dem Rundschüttler.

6. Überführung der inkubierten 30 ml-Ansätze in 50 ml-Probengefäße zur Zellernte.

7. Zentrifugation der Probengefäße bei 8000 Umdrehungen/min für 8 min.

8. Resuspension des Pellets mit 20 ml Lysepuffer zur Trennung von Proteinen und Fetten der Milch von den Zellen und anschließende Inkubation für 10 min bei RT.

9. Zentrifugation für 8 min bei 8000 Umdrehungen/min.

10. Resuspension der pelletierten Zellen in 600 $\mu$l 1 x PBS und Überführung in ein 2 ml-Reaktionsgefäß. Spülen des 50 ml Probengefäßes mit 600 $\mu$l 1 x PBS.

11. Zentrifugation der Reaktionsgefäße bei 14000 Umdrehungen/min für 6min.

12. Verwerfen des Überstandes und Resuspension der Zellen in 1 ml 1 x PBS.

13. Zentrifugation der Eppendorf-Reaktionsgefäße bei 14000 Umdrehungen/min für 6min.

14. Verwerfen des Überstandes und vollständige Resuspension des Pellets in 450 $\mu$l 1 x PBS.

15. Zugabe von 1350 $\mu$l einer frisch aufgetauten Paraformaldehydlösung.

16. Inkubation für 1 Stunde bei 4°C.

17. Überführung von je 800 μl in zwei 1,5 ml-Eppendorf-Reaktionsgefäße für die anschließende Hybridisierung mit "Salm63-Cy3" und "nonSalm-Cy3".

18. Zentrifugation bei 14000 Umdrehungen/ min für 6min.

19. Abnehmen und Verwerfen des Überstandes und vollständige Resuspension des Pellets in 1 ml 1 x PBS.

20. Zentrifugation bei 14000 Umdrehungen/ min für 6min.

21. Abnehmen und Verwerfen des Überstandes (Pellet verbleibt im Cap).

B. Hybridisierung

Laborausstattung:

- Tischzentrifuge (14000 Umdrehungen / Minute)
- Fluorometer (Kontron Instruments SFM 25)
- Heizblock und Wasserbad oder zwei Heizblöcke (48°C und 80 °C)
- Hybridisierungsofen (46°C)

Materialien

- Formamid (Merck, Darmstadt)
- Sonden-Arbeitslösungen zu je 50 ng/μl: 25 μl "Salm63-Cy3", 25 μl "nonSalm-Cy3"

Ablösepuffer (2 ml):

0.01 M Tris/HCl    pH 9,0

Hybridisierungspuffer (2 ml mit 40% Formamidgehalt):

| | |
|---|---|
| 5 M NaCl | 360 μl |
| 1 M Tris/HCl pH8,0 | 40 μl |
| Formamid | 800 μl |
| 10 % (w/v) SDS | 2 μl |
| $H_2O_{bidest}$ | ad 2 ml |

Waschpuffer (2ml):

| | |
|---|---|
| 5 M NaCl | 18,4 μl |
| 1 M Tris/HCl pH 8,0 | 40 μl |
| 0,5 M EDTA pH 8,0 | 20 μl |
| 10% (w/v) SDS | 2 μl |
| $H2O_{bidest}$ | ad 2 ml |

Durchführung:

1. Vorwärmen des Hybridisierungspuffers auf 48 °C im Wasserbad oder im Heizblock für ca. 20 min.

2. Überführung der Eppendorf-Reaktionsgefäße (mit dem zurückgebliebenen Zellpellet) auf den Heizblock (auf 80°C vorgeheizt) und Inkubation für 5 min bei 80 °C.

3. Nach der Inkubation bei 80 °C für 5 min Zugabe von 160 $\mu$l des vorgewärmten Hybridisierungspuffers in jedes Eppendorf-Reaktionsgefäß.

4. Zugabe von je 3,0 $\mu$l der frisch aufgetauten Sonden-Arbeitslösung "Salm63-Cy3" zu den zwei Parallelansätzen.

5. Zugabe von je 3,0 $\mu$l der frisch aufgetauten Sonden-Arbeitslösung "nonSalm-Cy3" zu den beiden verbleibenden Parallelansätzen.

6. Wirlen (Vortexen) der Eppendorf-Reaktionsgefäße für 10 Sekunden und kurzes (2 Sekunden) Abzentrifugieren mittels einer kleinen Tischzentrifuge.

7. Inkubation der Eppendorf-Reaktionsgefäße für 2 Stunden bei 46 °C im Hybridisierungsofen oder im Wasserbad bzw. Heizblock (Hybridisierung der Sonde mit der Zielsequenz unter stringenten Bedingungen).

8. Vorwärmen des Waschpuffers auf 48 °C im Wasserbad oder im Heizblock.

9. Nach der Inkubation für 2 Stunden bei 46 °C Zentrifugation der Eppendorf-Reaktionsgefäße bei 14000 Umdrehungen/ min für 6min.

10. Vollständiges Abnehmen und Verwerfen des Überstandes mit einer 200 $\mu$l-Pipette ohne das Pellet zu berühren.

11. Zugabe von 180 $\mu$l vorgewärmtem Waschpuffer, Wirlen der Eppendorf-Reaktionsgefäße für 5 Sekunden und Überführung ins Wasserbad bei 48 °C für 20 Minuten (Entfernen der nicht gebundenen Sondenmoleküle unter stringenten Bedingungen.

12. Vorwärmen von ca. 1,4 ml Ablösepuffer auf 80 °C im Heizblock.

13. Nach Inkubation bei 48 °C für 20 min Zentrifugation der Eppendorf-Reaktionsgefäße bei 14000 Umdrehungen/ min für 6min.

14. Vollständiges Abnehmen und Verwerfen des Überstandes mit einer 200 $\mu$l-Pipette ohne das Pellet zu berühren.

15. Zugabe von 130 $\mu$l vorgewärmtem Ablösepuffer, Wirlen der Eppendorf-Reaktionsgefäße für 5 Sekunden und Überführung auf den Heizblock auf 80 °C für 15 Minuten (Ablösung der Sonde von der Zielsequenz).

16. Nach Inkubation bei 80°C für 15 min Zentrifugation der Eppendorf-Reaktionsgefäße bei 14000 Umdrehungen/ min für 6min.

17. Vorsichtige Überführung des Überstandes mit einer 200 $\mu$l-Pipette ohne das Pellet zu berühren in neue 1,5 ml-Reaktionsgefäße und anschließend Lagerung auf Eis im Dunkeln bis zur Vermessung.

18. Fluorometer anschalten und die Wellenlängen auf 550 nm zur Anregung ("Excitation wavelength") und auf 570 nm ("Emission wavelenght") zur Messung der Emission des Cy3-Farbstoffes einstellen.

19. High Voltage Level auf die gewünschte Empfindlichkeit einstellen (400 bis 800 HVL).

20. 108 $\mu$l Ablösepuffer in eine Präzisionsglaskuevette für die Fluorometrie füllen.

21. Fluorometer auf Null stellen ("Autozero").

22. Eppendorf-Reaktionsgefäße kurz vor der Vermessung 5 Sekunden in der Hand auf Raumtemperatur vorwärmen.

23. Einfüllen des die abgelöste Sonde enthaltenden Ablösepuffers in die Präzisionsglaskuevette für die Fluorometrie und Vermessung des Signals.

24. Ablesen des Signals nach 10 Sekunden, da nach dem Öffnen der Abdeckung des Lichtkanals das Signal nach 5 bis 8 Sekunden stabil ist.

25. Fluoreszenzwert der untersuchten Milchprobe = Fluoreszenzwert der mit "Salm63-Cy3" hybridisierten Milchprobe abzüglich dem Fluoreszenzwert der mit "nonSalm-Cy3" hybridisierten Milchprobe.

BEISPIEL 3

Anwendung der Fasi-FISH-Technik zur relativen Quantifizierung von Bakterienpopulationen in Belebtschlamm (am Beispiel PPx3)

A. Zellfixierung:

Laborausstattung:

- 2 ml-Reaktionsgefäße
- Tischzentrifuge (14000 Umdrehungen / Minute)

Zur PFA-Zellfixierung verwendete Lösungen:
• PBS-Stammlösung ($Na_xPO_4$)

$$200 \text{ mM } NaH_2PO_4$$
$$200 \text{ mM } Na_2HPO_4 \quad pH\ 7,2 - 7,4$$

• 3 x PBS-Lösung

$$390 \text{ mM } NaCl$$
$$30 \text{ mM } Na_xPO_4 \text{ (PBS-Stammlösung)} \quad pH\ 7,2 - 7,4$$

• 1 x PBS-Lösung

$$130 \text{ mM } NaCl$$
$$10 \text{ mM } Na_xPO_4 \text{ (PBS-Stammlösung)} \quad pH\ 7,2 - 7,4$$

• 4% Paraformaldehydlösung (PFA):
Herstellbar durch Zugabe von 3 g Paraformaldehyd zu 30 ml auf 60°C erhitztem $H_2O_{bidest.}$; tropfenweise Zugabe von 1 M NaOH bis zum vollständigen Lösen des Paraformaldehyds; anschließend Hinzufügen von 16,6 ml 3 x PBS, Abkühlung der
Lösung auf ca. 20°C; Einstellen des pH-Wertes mit 1 M HCl auf 7,2 - 7,4; Sterilfiltration der fertigen PFA-Lösung über einen $0,2\mu m$-Filter (MILLIPORE, Eschborn). Die Lösung kann bei 4°C für ca. eine Woche aufbewahrt werden; Einfrieren über mehrere Monate ist ebenfalls möglich.

Durchführung:

1. Zugabe von drei Teilen 4 %iger Paraformaldehydlösung zu einem Teil frisch gezogener Belebtschlammprobe (z.B. 30 ml 4 %ige Paraformaldehydlösung zu 10 ml Belebtschlamm).

2. Inkubation für 3 Stunden bei 4°C.

3. Zentrifugation bei 8000 Umdrehungen/ min für 6min.

4. Verwerfen des Überstandes und vollständige Resuspension des Pellets in 1 ml 1 x PBS.

5. Zentrifugation bei 8000 Umdrehungen/ min für 6min.

6. Verwerfen des Überstandes (Pellet verbleibt im Cap).

7. Resuspension des Pellets mit 250 µl 1 x PBS.

8. Zugabe von 250 µl -18 °C-kaltem Ethanol.

9. Gründlich wirlen und gegebenenfalls bei -18 °C lagern (fixierte Belebtschlammprobe).

B. Hybridisierung

Laborausstattung:

- Tischzentrifuge (14000 Umdrehungen / Minute)
- Fluorometer
- 1 Heizblock und 1 Wasserbad, oder 2 Heizblöcke
- Hybridisierungsofen

Materialien:

- Eppendorf 1,5 ml Reaktionsgefäße
- Formamid (Merck, Darmstadt)
- Sonden-Arbeitslösungen zu je 50 ng / µl:

| | | |
|---|---|---|
| 25 µl "PPx3 655-Cy3": | | 5'- ACC CTC CTC TCC CGG TCT -3' |
| alternativ zu PPx3 655-Cy3 kann PPx3 1428-Cy3 verwendet werden " PPx3 1428-Cy3 | | 5'- TGG CCC ACC GGC TTC GGG -3' |
| 25 µl "Non15-Cy3": | | 5'- GCT AAC TAC TTC TGG AGC - 3' |
| 25 µl "Non15-Cy5": | | 5'- GCT AAC TAC TTC TGG AGC - 3' |
| 25 µl "3xEUB-Cy3": | I | 5'- GCT GCC TCC CGT AGG AGT - 3' |
| | II | 5'- GCA GCC ACC CGT AGG TGT - 3' |
| | III | 5'- GCT GCC ACC CGT AGG TGT - 3' |
| 25 µl "3xEUB-Cy5" | I | 5'- GCT GCC TCC CGT AGG AGT - 3' |
| | II | 5'- GCA GCC ACC CGT AGG TGT - 3' |
| | III | 5'- GCT GCC ACC CGT AGG TGT - 3' |

Ablösepuffer (2 ml):
0,01 M Tris/HCl pH 9,0
Hybridisierungspuffer: (2 ml mit 20% Formamidgehalt):

| | |
|---|---|
| 5 M NaCl | 360 µl |
| 1 M Tris/HCl pH8,0 | 40 µl |
| Formamid | 400 µl |
| 10 % (w/v) SDS | 2 µl |
| $H_2O_{bidest}$ | ad 2 ml |

Waschpuffer (2ml):

| | |
|---|---|
| 5 M NaCl | 86 $\mu$l |
| 1 M Tris/HCl pH 8,0 | 40 $\mu$l |
| 0,5 M EDTA pH 8,0 | 20 $\mu$l |
| 10% (w/v) SDS | 2 $\mu$l |
| $H2O_{bidest}$ | ad 2 ml |

Ansatz (genaues Vorgehen: siehe "Durchführung"):

1.2x definiertes Volumen fixierte Belebtschlammprobe (10 bis 100 $\mu$l) + 3xEub-Cy3
2.2x definiertes Volumen fixierte Belebtschlammprobe + 3xEub-Cy5
3.2x definiertes Volumen fixierte Belebtschlammprobe + Non15-Cy3
4.2x definiertes Volumen fixierte Belebtschlammprobe + Non15-Cy5
5.2x definiertes Volumen fixierte Belebtschlammprobe + 3xEub-Cy5 + PPx3 655-Cy3

Durchführung:

1. Vorwärmen des Hybridisierungspuffers auf 48 °C im Wasserbad oder im Heizblock für ca. 20 min.

2. Überführung eines definiertes Volumens PFA-fixierter Belebtschlammprobe (10 bis 100 $\mu$l) in Eppendorf-Reaktionsgefäße

3. Zentrifugation bei 14000 Umdrehungen/min für 6 min.

4. Abnehmen und Verwerfen des Überstandes mit einer 200 $\mu$l-Pipette ohne das Pellet zu berühren.

5. Überführung der Eppendorf-Reaktionsgefäße (mit dem zurückgebliebenen Zellpellet) auf den Heizblock (auf 80 °C vorgeheizt) und Inkubation für 5 min bei 80 °C.

6. Nach der Inkubation bei 80 °C für 5 min Zugabe von 80 $\mu$l des vorgewärmten Hybridisierungspuffers in jedes Eppendorf-Reaktionsgefäß.

7. Zugabe von je 2,5 $\mu$l der entsprechenden frisch aufgetauten Sonden-Arbeitslösung zu den Belebtschlamm-Ansätzen (siehe unter "Ansatz").

8. Wirlen der Eppendorf-Reaktionsgefäße für 10 Sekunden und kurzes (2 Sekunden) Abzentrifugieren mittels einer kleinen Tischzentrifuge.

9. Inkubation der Eppendorf-Reaktionsgefäße für 2 Stunden bei 46 °C im Hybridisierungsofen oder im Wasserbad bzw. Heizblock (Hybridisierung der Sonde mit der Zielsequenz unter stringenten Bedingungen).

10. Vorwärmen des Waschpuffers auf 48 °C im Wasserbad oder im Heizblock.

11. Nach der Inkubation für 2 Stunden bei 46 °C Zentrifugation der Eppendorf-Reaktionsgefäße bei 14000 Umdrehungen/ min für 6min.

12. Vollständiges Abnehmen und Verwerfen des Überstandes mit einer 200 $\mu$l-Pipette ohne das Pellet zu berühren.

13. Zugabe von 100 $\mu$l vorgewärmtem Waschpuffer, Wirlen der Eppendorf-Reaktionsgefäße für 5 Sekunden und Überführung ins Wasserbad bei 48 °C für 20 Minuten (Entfernen der nicht gebundenen Sondenmoleküle unter stringenten Bedingungen).

14. Vorwärmen des Ablösepuffers auf 80 °C im Heizblock.

15. Nach Inkubation bei 48 °C für 20 min Zentrifugation der Eppendorf-Reaktionsgefäße bei 14000 Umdrehungen/min für 6min.

16. Vollständiges Abnehmen und Verwerfen des Überstandes mit einer 200 $\mu$l-Pipette ohne das Pellet zu berühren.

17. Zugabe von 110 $\mu$l vorgewärmtem Ablösepuffer, Wirlen der Eppendorf-Reaktionsgefäße für 5 Sekunden und Überführung auf den Heizblock auf 80 °C für 15 Minuten (Ablösung der Sonde von der Zielsequenz).

18. Nach Inkubation bei 80°C für 15 min Zentrifugation der Eppendorf-Reaktionsgefäße bei 14000 Umdrehungen/min für 6min.

19. Vorsichtige Überführung des Überstandes mit einer 200 $\mu$l-Pipette ohne das Pellet zu berühren in neue 1,5 ml-Reaktionsgefäße und anschließend Lagerung auf Eis im Dunkeln bis zur Vermessung.

20. Fluorometer anschalten.

21. Für die Vermessung der Cy3-markierten Sonden Wellenlänge zur Anregung auf 550 nm ("Excitation wavelength") und zur Messung der Emission auf 570 nm ("Emission wavelenght") einstellen.

22. Für die Vermessung der Cy5-markierten Sonden Wellenlänge zur Anregung auf 644 nm ("Excitation wavelength") und zur Messung der Emission auf 659 nm ("Emission wavelenght") einstellen.

23. Bei Ansätzen mit beiden Farbstoffen (PPx3-Cy3 und 3xEub-Cy5) wird stets zuerst der Cy5-Wert und danach der Cy3-Wert bestimmt (wegen Ausbleicheffekten).

24. High Voltage Level auf die gewünschte Empfindlichkeit einstelllen (400 bis 800 HVL).

25. 108 $\mu$l Ablösepuffer in eine Präzisionsglaskuevette für die Fluorometrie füllen.

26. Fluorometer auf Null stellen ("Autozero").

27. Eppendorf-Reaktionsgefäße kurz vor der Vermessung 5 Sekunden in der Hand auf Raumtemperatur vorwärmen.

28. Einfüllen des die abgelöste Sonde enthaltenden Ablösepuffers in die Präzisionsglaskuevette für die Fluorometrie und Vermessung des Signals.

29. Ablesen des Signals nach 10 Sekunden, da nach dem Öffnen der Abdeckung des Lichtkanals das Signal nach 5 bis 8 Sekunden stabil ist.

Berechnung des relativen Anteils der PPx3-Zellen im untersuchten Belebtschlamm:

$$\frac{\text{Durchschnittlicher Meßwert der Belebtschlammprobe (BP) mit 3xEub-Cy}}{\text{Durchschnittlicher Meßwert der BP mit 3xEub-Cy5}} = \text{Korrekturfaktor}$$

Relativer Anteil der PPx3 Zellen in Prozent =

$$\frac{(\text{Meßwert BP mit PPX3-Cy3} - \text{durchschnittl. Meßwert BP mit Non15-Cy3}) \times 100}{(\text{Meßwert BP mit 3xEub-Cy5} - \text{durchschnittl. Meßwert BP mit Non15-Cy5}) \times}$$

Korrekturfaktor

**Patentansprüche**

1.  Verfahren zum Nachweisen von Mikroorganismen in einer Probe mittels einer Nukleinsäuresonde, umfassend die folgenden Schritte:

    a) Fixieren der in der Probe enthaltenen Mikroorganismen;
    b) Inkubieren der fixierten Mikroorganismen mit nachweisbaren Nukleinsäuresondenmolekülen;
    c) Entfernen nicht hybridisierter Nukleinsäuresondenmoleküle,
    d) Ablösen der hybridisierten Nukleinsäuresondenmoleküle ohne Einsatz von Formamid, und
    e) Detektieren der abgelösten Nukleinsäuresondenmoleküle.

2.  Verfahren nach Anspruch 1, worin die abgelösten Nukleinsäuresondenmoleküle in Schritt e) zusätzlich quantifiziert werden.

3.  Verfahren nach Anspruch 1 oder 2, worin die in Schritt d) verwendete Ablöselösung ausgewählt ist aus der Gruppe bestehend aus Wasser, gepuffertem Wasser, DMSO und SSC.

4.  Verfahren nach Anspruch 3, worin es sich bei der Ablöselösung um 0,001-1,0 M Tris/HCl, pH 9,0 +/- 2,0 handelt.

5.  Verfahren nach Anspruch 3 oder 4, worin es sich bei der Ablöselösung um 0,01 M Tris/HCl, pH 9,0 +/- 2,0 handelt.

6.  Verfahren nach einem der vorangehenden Ansprüche, worin Schritt d) bei einer Temperatur von 50-100°C erfolgt.

7.  Verfahren nach einem der vorangehenden Ansprüche, worin Schritt d) bei einer Temperatur von unter 100°C erfolgt.

8.  Verfahren nach einem der vorangehenden Ansprüche, worin Schritt d) bei einer Temperatur von ungefähr 80°C durchgeführt wird.

9. Verfahren nach einem der vorangehenden Ansprüche, worin die Nukleinsäuresonde komplementär zu einer chromosomalen oder episomalen DNA, einer mRNA oder rRNA eines nachzuweisenden Mikroorganismus ist.

10. Verfahren nach einem der vorangehenden Ansprüche, worin die Nukleinsäuresonde kovalent mit einem detektierbaren Marker verbunden ist.

11. Verfahren nach Anspruch 10, worin der detektierbare Marker ausgewählt ist aus der Gruppe der folgenden Marker:

    a) Fluoreszenzmarker,
    b) Chemolumineszenzmarker.
    c) radioaktiver Marker,
    d) enzymatisch aktive Gruppe,
    e) Hapten,
    f) durch Hybridisierung nachweisbare Nukleinsäure.

12. Verfahren nach einem der vorangehenden Ansprüche, worin der Mikroorganismus ein einzelliger Mikroorganismus ist.

13. Verfahren nach einem der vorangehenden Ansprüche, worin der Mikroorganismus eine Hefe, ein Bakterium, eine Alge oder ein Pilz ist.

14. Verfahren nach Anspruch 13, wobei der Mikroorganismus der Gattung *Salmonella* angehört.

15. Verfahren nach einem der vorangehenden Ansprüche, wobei die Probe eine Umweltprobe und aus Wasser, Boden oder Luft entnommen ist.

16. Verfahren nach einem der Ansprüche 1 bis 14, wobei die Probe eine Lebensmittelprobe ist.

17. Verfahren nach Anspruch 16, wobei die Probe aus Milch oder Milchprodukten, Trinkwasser, Getränken, Backwaren oder Fleischwaren entnommen ist.

18. Verfahren nach einem der Ansprüche 1 bis 14, wobei die Probe eine medizinische Probe ist.

19. Verfahren nach Anspruch 18, wobei die Probe aus Gewebe, Sekreten oder Stuhl gewonnen ist.

20. Verfahren nach einem der Ansprüche 1 bis 14, wobei die Probe aus Abwasser gewonnen ist.

21. Verfahren nach Anspruch 20, wobei die Probe aus Belebtschlamm, Faulschlamm oder anaerobem Schlamm gewonnen ist.

22. Verfahren nach einem der Ansprüche 1 bis 14, wobei die Probe aus einem Biofilm gewonnen wird.

23. Verfahren nach Anspruch 22, wobei der Biofilm aus einer industriellen Anlage gewonnen wird, bei der Abwasserreinigung gebildet wurde oder ein natürlicher Biofilm ist.

24. Verfahren nach einem der Ansprüche 1 bis 14, wobei die Probe einem pharmazeutischen oder kosmetischen Produkt entnommen ist.

25. Kit zur Durchführung des Verfahrens nach einem der vorangehenden Ansprüche, enthaltend

    a) mindestens einen Hybridisierungspuffer,
    b) mindestens eine Nukleinsäuresonde,
    b1) zum spezifischen Nachweis eines Mikroorganismus,
    b2) zum Durchführen einer Negativkontrolle.

26. Kit nach Anspruch 25, enthaltend mindestens eine spezifische Sonde zum Nachweis von Bakterien der Gattung *Salmonella.*

**27.** Kit nach Anspruch 26, enthaltend die Nukleinsäuresonden

Salm63: 5'-TCGACTGACTTCAGCTCC-3'
und
NonSalm: 5'-GCTAACTACTTCTGGAGC-3'

oder eine Nukleinsäuresonde, die sich von Salm63 und/oder NonSalm durch eine Deletion und/oder Addition unterscheidet, wobei die Fähigkeit dieser Sonde, mit Salmonella-spezifischer Nukleinsäure zu hybridisieren, erhalten bleibt, oder eine Nukleinsäure, die mit den zuvor genannten Nukleinsäuren hybridisieren kann.

**Claims**

**1.** A method for the detection of microorganisms in a sample by means of a nucleic acid probe, comprising the following steps:

a) fixing the microorganisms contained in the sample;
b) incubating the fixed microorganisms with detectable nucleic acid probe molecules;
c) removing non-hybridized nucleic acid probe molecules;
d) releasing the hybridized nucleic acid probe molecules without the use of formamide, and
e) detecting the released nucleic acid probe molecules.

**2.** The method according to claim 1, wherein the released nucleic acid probe molecules are additionally quantified in step e).

**3.** The method according to claim 1 or 2, whereby the releasing solution used in step d) is selected from the group consisting of water, buffered water, DMSO and SSC.

**4.** The method according to claim 3, whereby the releasing solution is 0.001 - 1.0 M Tris/HCl, pH 9.0 +/- 2.0.

**5.** The method according to claim 3 or 4, whereby the releasing solution is 0.01 M Tris/HCl, pH 9.0 +/- 2.0.

**6.** The method according to any of the preceding claims, whereby step d) is performed at a temperature of 50 - 100°C.

**7.** The method according to any of the preceding claims, whereby step d) is performed at a temperature of below 100 °C.

**8.** The method according to any of the preceding claims, whereby step d) is performed at a temperature of about 80 °C.

**9.** The method according to any of the preceding claims, whereby the nucleic acid probe is complementary to a chromosomal or episomal DNA, an mRNA or rRNA of a microorganism to be detected.

**10.** The method according to any of the preceding claims, whereby the nucleic acid probe is covalently linked to a detectable marker.

**11.** The method according to claim 10, whereby the detectable marker is selected from the group comprising the following markers:

a) fluorescence marker,
b) chemiluminescence marker,
c) radioactive marker,
d) enzymatically active group
e) hapten,
f) a nucleic acid detectable by hybridization.

**12.** The method according to any of the preceding claims, whereby the micro organism is a unicellular microorganism.

**13.** The method according to any of the preceding claims, whereby the microorganism is a yeast, a bacterium, an algae or a fungus.

**14.** The method according to claim 13, whereby the microorganism is a member of the genus *Salmonella.*

**15.** The method according to any of the preceding claims, whereby the sample is an environmental sample taken from water, soil or air.

**16.** The method according to any of claim 1 to 14, whereby the sample is a foodstuff sample.

**17.** The method according to claim 16, whereby the sample is taken from milk or dairy products, drinking water, beverages, bakery products or meat products.

**18.** The method according to any of claims 1 to 14, whereby the sample is a medicinal sample.

**19.** The method according to claim 18, whereby the sample is taken from tissue, secreta or faeces.

**20.** The method according to any of claims 1 to 14, whereby the sample is taken from sewage.

**21.** The method according to claim 20, whereby the sample is taken from activated sludge, digested sludge or anaerobic sludge.

**22.** The method according to any of claims 1 to 14, whereby the sample is obtained from a biofilm.

**23.** The method according to claim 22, whereby the biofilm is obtained from an industrial plant, is formed in the treatment of waste water or is a natural biofilm.

**24.** The method according to any of claims 1 to 14, whereby the sample is taken from a pharmaceutical or cosmetic product.

**25.** Kit for performing the method according to any of the preceding claims, comprising

       a) at least one hybridization buffer,
       b) at least one nucleic acid probe,
       b1) for the specific detection of a microorganism,
       b2) for performing a negative control.

**26.** The kit according to claim 25, containing at least one specific probe for the detection of bacteria of the genus *Salmonella.*

**27.** Kit according to claim 26, containing the nucleic acid probes

       Salm63: 5'-TCGACTGACTTCAGCTCC-3'
       and
       NonSalm: 5'-GCTAACTACTTCTGGAGC-3'

or a nucleic acid probe which differs from Salm63 and/or NonSalm by deletion and/or addition, whereby the capacity of said probe to hybridize to Salmonella-specific nucleic acid is maintained, or a nucleic acid which may hybridize with any of the aforementioned nucleic acids.

**Revendications**

**1.** Procédé pour la détection de micro-organismes dans un échantillon au moyen d'une sonde d'acides nucléiques, comprenant les étapes suivantes :

       a) fixer les micro-organismes contenus dans l'échantillon ;
       b) incuber les micro-organismes définis avec des molécules de sonde d'acides nucléiques détectables ;
       c) éliminer les molécules de sonde d'acides nucléiques non hybridées ;
       d) séparer les molécules de sonde d'acides nucléiques hybridées sans utiliser de formamide, et
       e) détecter les molécules de sonde d'acides nucléiques séparées.

**2.** Procédé selon la revendication 1, où les molécules de sonde d'acides nucléiques séparées sont en outre quantifiées à l'étape e).

**3.** Procédé selon la revendication 1 ou la revendication 2, où la solution de séparation mise en oeuvre à l'étape d) est choisie dans le groupe composé d'eau, d'eau tamponnée, de DMSO et de SSC.

**4.** Procédé selon la revendication 3, où la solution de séparation est de 0,001 à 1,0 M de Tris/HCl, pH 9,0 +/- 2,0.

**5.** Procédé selon la revendication 3 ou 4, où la solution de séparation est de 0,01 M de Tris/HCl, pH 9,0 +/- 2,0.

**6.** Procédé selon l'une des revendications précédentes, où l'étape d) est exécutée à une température comprise entre 50 et 100°C.

**7.** Procédé selon l'une des revendications précédentes, où l'étape d) est exécutée à une température inférieure à 100°C.

**8.** Procédé selon l'une des revendications précédentes, où l'étape d) est exécutée à une température de 80°C environ.

**9.** Procédé selon l'une des revendications précédentes, où la sonde d'acides nucléiques est complémentaire d'un ADN chromosomique ou épisomal, d'un ARN messager ou d'un ARN ribosomique d'un micro-organisme à détecter.

**10.** Procédé selon l'une des revendications précédentes, où la sonde d'acides nucléiques est liée de façon covalente avec un marqueur détectable.

**11.** Procédé selon la revendication 10, où le marqueur détectable est choisi dans le groupe des marqueurs suivants :

a) marqueur fluorescent,
b) marqueur chimioluminescent,
c) marqueur radioactif,
d) groupe enzymatique actif,
e) haptène,
f) acide nucléique détectable par hybridation.

**12.** Procédé selon l'une des revendications précédentes, où le micro-organisme est un micro-organisme monocellulaire.

**13.** Procédé selon l'une des revendications précédentes, où le micro-organisme est une levure, une bactérie, une algue ou un champignon.

**14.** Procédé selon la revendication 13, où le micro-organisme est du genre *Salmonella.*

**15.** Procédé selon l'une des revendications précédentes, où l'échantillon est un échantillon de l'environnement et prélevé à partir de l'eau, du sol ou de l'air.

**16.** Procédé selon l'une des revendications 1 à 14, où l'échantillon est un échantillon de produit alimentaire.

**17.** Procédé selon la revendication 16, où l'échantillon est prélevé à partir de lait ou de produits laitiers, de l'eau potable, de boissons, de produits de pâtisserie ou de produits carnés.

**18.** Procédé selon l'une des revendications 1 à 14, où l'échantillon est un échantillon médical.

**19.** Procédé selon la revendication 18, où l'échantillon est extrait de tissus, de sécrétions, ou de selles.

**20.** Procédé selon l'une des revendications 1 à 14, où l'échantillon est extrait d'eaux usées.

**21.** Procédé selon la revendication 20, où l'échantillon est extrait de boue activée, de boue putréfiée ou de boue anaérobie.

**22.** Procédé selon l'une des revendications 1 à 14, où l'échantillon est extrait d'un biofilm.

23. Procédé selon la revendication 22, où le biofilm est obtenu à partir d'une installation industrielle, formé par épuration des eaux usées, ou est un biofilm naturel.

24. Procédé selon l'une des revendications 1 à 14, où l'échantillon est prélevé d'un produit pharmaceutique ou cosmétique.

25. Kit pour l'exécution du procédé selon l'une des revendications précédentes, comprenant

    a) au moins un tampon d'hybridation,
    b) au moins une sonde d'acides nucléiques,
    b1) pour la détection d'un micro-organisme spécifique,
    b2) pour l'exécution d'un contrôle négatif.

26. Kit selon la revendication 25, comprenant au moins une sonde spécifique pour la détection de bactéries du genre *Salmonella.*

27. Kit selon la revendication 26, comprenant les sondes d'acides nucléiques

    Salm63 : 5'-TCGACTGACTTCAGCTCC-3'
    et
    NonSam : 5'-GCTAACTACTTCTGGAGC-3'

ou une sonde d'acides nucléiques se distinguant de Salm63 et/ou de NonSalm par une délétion et/ou une addition, la capacité de ladite sonde à s'hybrider avec de l'acide nucléique spécifique à la Salmonella étant maintenue, ou un acide nucléique, apte à s'hybrider avec les acides nucléiques susmentionnés.

Abb. 1

# Abb. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19936875

**In der Beschreibung aufgeführte Nicht-Patentliteratur**

- **AMANN et al.** *J. Bacteriol.,* 1990, vol. 172, 762
- **WOESE.** *Microbiol.,* 1987, vol. 51, 221
- **DE LONG et al.** *Science,* 1989, vol. 243, 1360
- **SNAIDR et al.** *Appl. Environ. Microbiol.,* 1997, vol. 63, 2884
- **WAGNER et al.** *Appl. Environ. Microbiol.,* 1993, vol. 59, 1520
- **BEIMFOHR et al.** *System Appl. Microbiol.,* 1993, vol. 16, 450
- **STAHL ; AMANN.** Nucleic Acid Techniques in Bacterial Systematics. John Wiley & Sons Ltd, 1991